# EUROPEAN PATENT SPECIFICATION

(11) **EP 0 331 959 B1**
(45) Date of publication and mention of the grant of the patent: **28.12.1994**
(21) Application number: 89102915.9
(22) Date of filing: 20.02.1989
(51) Int. Cl.: A61N 1/375

(54) **Bipolar filtered feedthrough terminal**
Bipolarer gefilterter Durchführungsanschluss
Raccordement de traversée bipolaire filtré

(30) Priority: 29.02.1988 US 161700
(43) Date of publication of application: 13.09.1989
(73) Proprietor: Pacesetter AB, 171 95 Solna (SE)
(72) Inventor: Truex, Buehl E., Glendora, CA 91740 (US)
(74) Representative: Rees, David Christopher

(56) References cited:
- EP-A- 0 052 879
- EP-A- 0 251 158
- DE-A- 2 809 886
- DE-A- 2 818 635
- US-A- 4 678 868
- US-A- 4 679 867

## Description

The invention relates generally to hermetically sealed feedthrough elements, and in particular to such an element utilizing a dual capacitor circuit for electromagnetic interference (EMI) protection for cardiac pacemakers.

### BACKGROUND OF THE INVENTION

The technology of cardiac pacemakers has reached a state of highly sophisticated system performance. The present generation of cardiac pacemakers uses microprocessors and related circuitry to sense and stimulate heart activity under a variety of physiological conditions. Currently available pacemakers can be programmed to control heart function in correcting or compensating for various cardiac abnormalities which may be encountered in individual patients. An extensive description of modern cardiac pacemaker technology is given in International Application No. PCT/US85/02010, entitled "Stimulated Heart Interval Measurement, Adaptive Pacer and Method of Operation," assigned to the assignee of the present invention. The disclosure of that application is incorporated herein by reference.

In brief, a cardiac pacemaker consists of a generator unit surgically implanted in the chest of a patient and connected to leads implanted in the heart. The heart leads are used both for sensing the electrical activity of the heart and for providing electrical stimulii to control the functioning of the heart. Connection of the heart leads to the pacemaker generator unit is made through hermetically sealed feedthrough terminals in the cap of the pacemaker.

It is desirable to eliminate electromagnetic interference from being fed into the leads entering the generator unit of a pacemaker. This can be done by using capacitive filtering. Previously, capacitive filtering for EMI protection was effected through utilization of discrete capacitors wired into the circuitry. The impedance of the leads going to the discrete components prevented the broad spectrum filtering that is ideally desired. Furthermore, some types of hermetic feedthrough elements involving capacitors for filtering can only be tested after final assembly, and if one part fails the test, the remainder of the assembly cannot be reworked or salvaged, thereby incurring substantial waste. It would be a significant advance in the technology of hermetically sealed feedthrough elements if one could be designed in such a way as to provide broad spectrum filtering of EMI and at the same time provide an economical and efficient unit for which testing of all component parts could be completed before committing to final assembly.

### SUMMARY OF THE INVENTION

In brief, arrangements in accordance with the present invention utilize a hermetically sealed feedthrough element with a dual capacitor circuit for electromagnetic interference protection for cardiac pacemakers. Novel capacitor and feedthrough structure results in broad-spectrum filtering and convenience in testing.

The purpose of the feedthrough element is to carry two circuit conductors from inside the pacemaker housing to connect to terminals into which the pacemaker leads are inserted. The feedthrough element of the invention is formed of a titanium body with a ceramic plug mounted at one end. The ceramic plug or end cap has the two conductors mounted in it, and is mounted in the titanium body to project slightly from one end, thereby providing a longer path length between the conductors and the titanium housing, which is ground, or reference potential. The end of the ceramic plug is also provided with a transverse slot located between the two conductors on the exterior side of the plug so as to provide a longer leakage path between the conductors. The ceramic end cap is sealed to one end of the titanium body with Kyroflex™. Into the other end of the titanium body is fitted an EMI capacitor in the form of a laminated disk comprising individual layers coated with thick conductive film to provide two sets of interleaved capacitive plates. One set consists of disk layers having openings about the two conductors and arranged to make contact with the outer circumferential surface of the end cap. The other set is circular with a circular film coating having a diameter less than the diameter of the end cap, with holes for two contact tubes which accommodate the conductor leads. This set of disk layers has alternate electrical connection of the conductive film layers with one or the other of the contact tubes. Two separate parallel-plate capacitors are thus formed which link the terminal leads with the titanium body ground.

Another important aspect of the invention is the way in which electrical connection is made between the outer circumference of the EMI capacitor, which is plated, and the inner diameter of the titanium body. Connection is made with a contact ring that starts out as a flat strip which is stamped along one edge by shear forming to develop a series of slightly rotated segments that project in alternate directions at both edges from the plane of the strip. The strip is then formed into a circle with a gap between the two ends. This contact ring is held around the capacitor disk and both are inserted into the titanium housing. The sharp edges of the rotated tabs on the contact ring make the desired contact between the inner surface of the titanium shell and the outer surface of the capacitor disk. The circuit connection is firmly established both mechanically and electrically. One advantage of this type of construction is that the capacitor disk can be tested as a unit before it is installed in the titanium body. After installation, the outer surface of the titanium body is resistance welded to the pacemaker housing and the outer ends of the two connecting tubes are welded to the corresponding conducting leads. The EMI protection provided covers an effective range from 27 MHz to 2.45 GHz.

### BRIEF DESCRIPTION OF THE DRAWINGS

The above and other objects, features and advantages of the present invention will be more apparent from the following more particular description thereof presented in conjunction with the accompanying drawings, wherein:
FIG. 1 is an exploded view of the bipolar filtered feedthrough terminal of the present invention;
FIG. 2A is an end view of the ceramic end cap of the terminal of FIG. 1 as seen from the outside of the feedthrough terminal;
FIG. 2B is a side view, partially broken away, of the end cap;
FIG. 3A is an end view of one contact tube of FIG. 1;
FIG. 3B is a partially broken away side view of the contact tube;
FIG. 3C is a sectional view along the line A-A in FIG. 3B after the vertical diameter has been slightly deformed;
FIGS. 4A-4C are plan views of three types of laminations of the EMI capacitor;
FIG. 5A is a schematic diagram of the two interleaved parallel-plate capacitors;
FIG. 5B is a schematic electrical diagram of the two separate capacitors formed by the laminations of the EMI capacitor;
FIG. 6A is a plan view of the sheer-formed metal strip from which the contact ring is made;
FIG. 6B is an elevational view of the sheer-formed metal strip of FIG. 6A;
FIG. 6C is a detailed view of the area marked A in FIG. 6B; and
FIG. 6D is a top view of the contact ring made from the sheer-formed metal strip shown in FIGS. 6A-6C.

### DESCRIPTION OF THE PREFERRED EMBODIMENTS

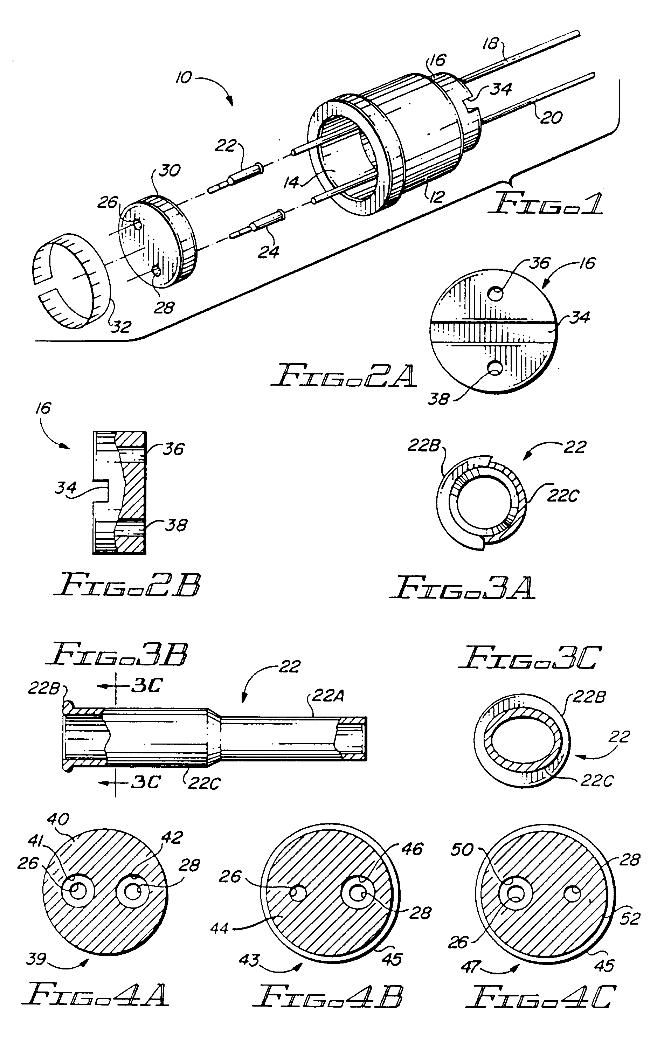

A bipolar filtered feedthrough terminal in accordance with the present invention is shown in the exploded view of FIG. 1. The feedthrough terminal 10 houses several components in a cylindrically symmetrical metal shell 12 having a central cylindrical bore 14 on its longitudinal axis. A ceramic end cap 16 is hermetically sealed into one end of the shell 12 with leads 18 and 20 extending through end cap 16 into the interior of shell 12. Leads 18 and 20 are hermetically sealed in holes through ceramic cap 16. Contact tubes 22 and 24 fitting onto the interior ends of leads 18 and 20 in turn fit through holes 26 and 28 of the EMI capacitor 30. EMI capacitor 30 comprises a series of laminations which consist of patterns of thick conducting films laid down on insulating substrates to form separate capacitors between contact tubes 22 and 24 and shell 12, which is maintained at a selected reference potential; e.g., ground. The different types of laminations and their order in forming EMI capacitor 30 are explained in detail below. A metal contact ring 32 fits around the outer circumference of EMI capacitor 30, which is metal plated, and inside the inner circumference of hole 14 in shell 12 to establish the common connection.

Shell 12 is preferably made of titanium metal. Ceramic end cap 16 is bonded into one end of the shell 12 with a material such as Kyroflex™, which is fired at about 1400°F. As shown in FIG. 1, ceramic end cap 16 is inserted only part way into shell 12, with roughly half of its thickness inside shell 12 and the other half outside shell 12. This partial insertion has the effect of increasing the resistive path length over the surface of the ceramic between leads 18 and 20 or between either lead and the shell 12, which is ground. The leakage resistance path between leads 18 and 20 is further increased by slot 34 in the exterior face of end cap 16.

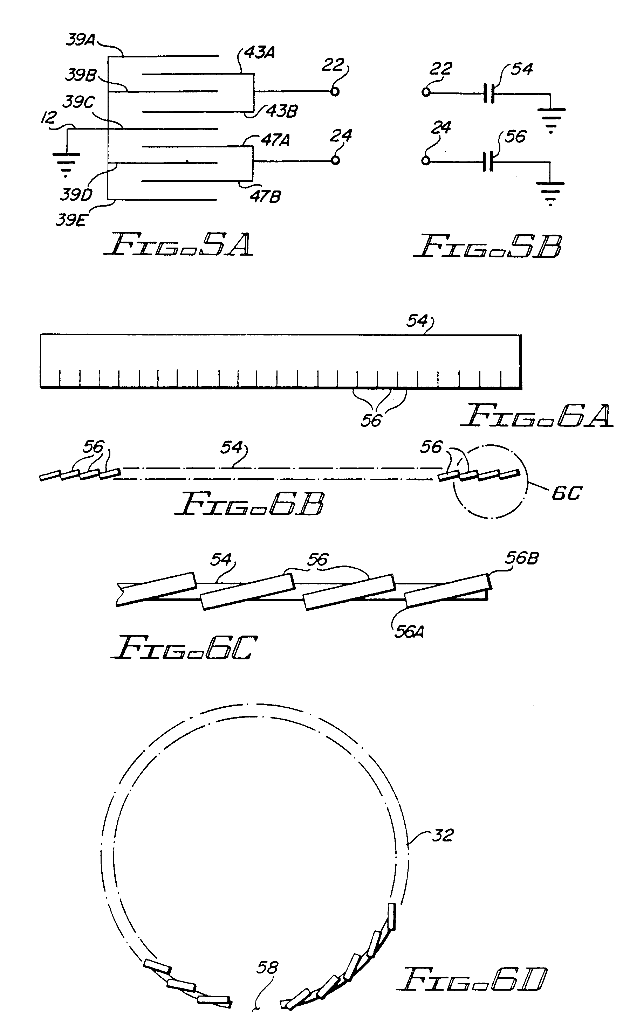

Ceramic end cap 16 is shown in more detail in FIGS. 2A and 2B. End cap 16 is made from an insulating ceramic such as alumina. Parallel feedthrough holes 36 and 38 to either side of slot 34 allow passage of leads 28 and 20 from the outside of end cap 16 to the interior shell 12. Leads 18 and 20 are hermetically sealed to end cap 16 with Kyroflex™.

FIGS. 3A and 3B show end and side views, respectively, of one of the contact tubes 22, 24. Contact tubes 22 and 24 are preferably made from seamless nickel 200 tube, with No. 1 annealed temper, having an outer diameter of 0.020 inch maximum and inner diameter 0.014 inch minimum. Contact tube 22 comprises an end portion 22a, a flange 22b, and an intervening body portion 22c. Body portion 22c has a larger diameter than end portion 22a. As shown in FIG. 3C, which is a sectional view across the line indicated in FIG. 3B, wherein body portion 22c is squeezed so that it assumes an elliptical cross section. The outer diameter of contact tube 22 is nominally 0.0225 inch to begin with, and is deformed out of round by about .004 inches. The end portions 22A and 24A of contact tubes 22 and 24 are inserted through corresponding holes 26 and 28 in EMI capacitor 30 until the ferrules 22B and 24B at the ends of the body portions 22C and 24C bottom on the cap. The body portions 22C and 24C of contact tubes 22 and 24 are designed to provide interference fits in holes 26 and 28 of EMI capacitor 30.

EMI capacitor 30 is a laminated disk which is punched out of a lamination made from individual insulating layers coated with thick conductive film to provide interleaved capacitive plates with differing shapes of film on successive layers. In FIGS. 4A-4C, the different types of laminations are shown. The first type of lamination, 39, has a layer of conducting film 40 entirely covering the substrate except for small circular islands 41 and 42 surrounding holes 26 and 28. The second type of lamination, 43, has a circular area 44 of conducting film which is slightly smaller in diameter than the underlying substrate 45, with a small uncovered island 46 surrounding hole 28. The third type of lamination, 47, is in reality the same as 43 but rotated through 180° in the plane of the disk. Lamination type 47 has its small uncovered island 50 surrounding hole 26, so that when contact tubes 22 and 24 are inserted through holes 26 and 28 in the lamination, circular area 52 of conducting film makes contact with tube 28 but not with tube 26. The opposite is true for lamination type 43. Insertion of contact tubes 22 and 24 through a lamination type 39 results in no electrical contact between the conductive film 40 and either tube.

FIG. 5A is a schematic electrical diagram of a bipolar capacitor disk 30 made up of nine laminations. Laminations 39a, 43a, 39b, 43b, and 39c form a parallel-plate capacitor 54 (FIG. 5B) between contact tube 22 and shell 12 (ground). A separate parallel-plate capacitor 56 is formed between contact tube 24 and shell 12 (ground) by laminations 39c, 47a, 39d, 47d, and 39e. The outer circumference of bipolar capacitor disk 30 is metal plated to provide electrical contact between all laminations of type 39 in the lamination stack. Contact between the plated outer periphery of bipolar capacitor disk 30 and the inner circumference of shell 12 is made through contact ring 32, which is shown in detail in FIGS. 6A-6D.

Contact ring 32 starts out as flat strip of metal 54 as shown in FIG. 6A. Metal strip 54 is stamped along one edge by sheer forming to develop a series of slightly rotated segments 56 that project in alternate directions at both edges from the plane of the strip 54. The slightly rotated tab-like segments 56 are shown in the elevation view of FIG. 6B, and in further detail in FIG. 6C. Strip 54 is then formed into a circular ring with a gap between the two ends, as shown in the top view of FIG. 6D. Contact ring 32 is placed around capacitor disk 30 and then both are inserted into the titanium housing 12. The tab-like segments 56 project in the outward direction away from shell 12 as contact ring 32 and capacitor disk 30 are being inserted together. The sharp edges 56A and 56B of the rotated segments 56 make the desired contact between the inner surface of shell 12 and the outer surface of capacitor disk 30, thus firmly establishing the integrity of the circuit connection.

One of the advantages of this type of construction is that the capacitor disk 30 can be tested as a unit before it is installed in the shell 12. After installation, the outer surface of the titanium body of shell 12 is resistance welded to the pacemaker housing. The outer ends of contact tubes 22 and 24 are also resistance welded to the corresponding conducting leads 18 and 20.

Although the capacitor disk 30 is represented in FIG. 5A as comprising nine separate laminations or conducting layers interleaved together, that is merely by way of example and other, equivalent configurations may be utilized as, for example, a different number of laminations having different areas making up the individual conducting layers. A preferred minimum value for the capacitance of the disk 30 is 2700 pf., but this may be increased as desired over a range up to 100%. The measurement of capacitance in determining the value is made at 1 KHz and 1.0 volts rms.

The capacitor disk 30 is manufactured and sold by U.S. Microtek Components, having an address of 11144 Penrose, Sun Valley, California. The dielectric characteristics of the capacitive disk 30 are in accordance with code X7R, a standard designation well-known to those skilled in the related art.

In one preferred embodiment of the invention, the disk 30 was nominally .050 inches thick with a nominal outside diameter of .114 inches. The holes 26, 28 were each .0230 inches in diameter, spaced .275 inches on opposite sides from the center of the disk 30 along a diameter thereof. The strip 54 from which the ring 32 is fabricated is nominally .050 inches wide by .370 inches long, cut from .003-inch stock. The edge portion of the strip 54 which is shear-formed to develop the tabs 56 is approximately .015 inches wide, and each tab 56 is twisted sufficiently to project approximately .002 inches from the planar surface of the strip 54.

Protection from EMI is provided over a spectrum extending from about 27 MHz to 2.45 GHz. The impedance of the leads which are present in conventional capacitive filtering devices using discrete capacitors is eliminated. Testing of the present feedthrough element 10 is more convenient because the capacitor disk 30 can be tested as a unit before it is installed in shell 12. Therefore, there is no loss of titanium shell components as there would be if the feedthrough element were found to fail after assembly, as is done in conventional filtering systems.

Although there has been described above one specific arrangement of a bipolar filtered feedthrough terminal for a pacemaker in accordance with the invention for the purpose of illustrating the manner in which the invention may be used to advantage, it will be appreciated that the invention is not limited thereto. Accordingly, any and all modifications, variations or equivalent arrangements which may occur to those skilled in the art should be considered to be within the scope of the invention as defined in the annexed claims.

## Claims

1. A bipolar filtered feedthrough terminal (10) comprising:
a cylindrical conductive tubular shell (12);
an insulating end cap (16) having first and second electrical leads (18,20) extending therethrough and hermetically sealed therein, said end cap (16) being hermetically sealed inside a first end of said shell (12);
first and second metallic contact tubes (22,24) mounted on ends of said leads (18,20) inside said shell (12); and
a laminated bipolar capacitor disk (30) fitting into a second end of said shell (12) with said first and second contact tubes (22,24) passing through said disk (30), said disk (30) providing separate capacitors (54,56) between said shell and respective said first and second contact tubes (22,24).

2. The terminal of claim 1 further including resilient conductive means (32) fitted between an outer periphery of said laminated disk (30) and an inner surface of said shell (12) for providing electrical connection between said capacitors (54,56) and said shell.

3. The feedthrough terminal of claim 1 wherein said bipolar capacitor disk (30) has a plated outer circumference and comprises a plurality of laminations (39,43,47), each said lamination comprising a thick conductive film (40) on an insulating substrate and having first and second tube holes (26,28) therethrough, there being at least two discrete types (39,43,47) of laminations classified according to the shape of the conductive film (40) thereon, with no two adjacent laminations (39,43,47) being of the same type.

4. The terminal of claim 3 wherein a first lamination type (39) comprises thick conductive film (40) extending to the peripheral edge of the lamination (39) but spaced from said tube holes (26,28), and a second lamination type (43,47) comprises thick conductive film (40) extending to the edge of only one of said tube holes (26,28) and spaced from the peripheral edge of the lamination (43,47).

5. The terminal of claim 4 wherein a pair of laminations of said second type (43,47) are arrayed on opposite sides of a lamination of said first type (39) with said pair (43,47) being oriented to make electrical contact with respective ones of said first and second contact tubes (22,24), thereby forming separate capacitors (56,58) between said respective first and second contact tubes (22,24) and said lamination of said first type (39).

6. The terminal of claim 4 wherein said laminations are arrayed to form interleaved parallelplace capacitors, one such capacitor (54) between said first contact tube (22) and said shell (12), and a second such capacitor (56) between said second contact tube (24) and said shell.

7. The terminal of claim 4 wherein said bipolar capacitor disk (30) comprises n laminations of the second type (43,47) and n+1 laminations of the first type (39) interleaved together with n/2 laminations of the second type (43,47) connected together to the first contact tube (22) and the remaining laminations of the second type (43,47) being connected together to the second contact tube (24), the laminations of the first type (39) being connected together to a common reference point.

8. The terminal of claim 2 wherein said resilient conductive means (32) comprises an unclosed ring (32) of a thin metallic strip (54) with a plurality of tabs (56) having edges protruding from the ring surfaces to establish contact between the peripheral metallic surface of the disk (30) and the inner surface of the shell (12).

9. The terminal of claim 8 wherein said tabs (56) are formed by a plurality of transverse cuts in the thin metallic strip along one edge thereof, each tab being twisted slightly with respect to an adjacent uncut portion of said strip contiguous with said tabe.

10. The terminal of claim 11 or 12 wherein said contact ring (32) comprises nickel having a thickness of about 0.003 inch, and each tab (56) is substantially square with a length equal to roughly 0.3 times the width of said strip (54).

11. The terminal of any of the claims 3 - 10 wherein each contact tube (22,24) comprises an end portion (22a) at a first end of said tube (12) having a first diameter, a flange (22b) at a second end of said tube, and a body portion (22c) between said end portion (22a) and said flange (22b), said body portion (22c) having a second diameter which is greater than said first diameter and less than the diameters of said first and second tube holes (26,28) to permit insertion therein.

12. The terminal of claim 11 wherein said body (22c) portion is deformed out of round to establish an interference fit when inserted into a corresponding tube hole (26,28).

## Patentansprüche

1. Ein bipolarer gefilterter Durchführungsanschluß (10) mit : einem zylindrischen leitenden rohrförmigen Gehäuse (12);
einer isolierenden Endkappe (16) mit ersten und zweiten elektrischen Leitern (18,20), die durch die Endkappe durchgeführt und hermetisch darin eingeschlossen sind, wobei die Endkappe (16) hermetisch innerhalb eines ersten Endes des Gehäuses (12) eingeschlossen ist;
ersten und zweiten metallischen Kontaktröhren (22, 24), die innerhalb des Gehäuses (12) auf den Enden der Leiter (18,20) angebracht sind; und
einer laminierten bipolaren Kondensatorscheibe (30), die in das zweite Ende des Gehäuses (12) paßt und durch die erste und zweiten Kontaktröhren hindurchgeführt sind, wobei durch die Scheibe (30) separate Kondensatoren (54,56) zwischen dem Gehäuse und den jeweils ersten und zweiten Kontaktröhren (22,24) erzeugt werden.

2. Anschluß nach Anspruch 1 weiterhin mit federnden leitenden Mitteln (32), die zwischen die äußere Peripherie der laminierten Scheibe (30) und eine innere Oberfläche des Gehäuses (12) passen, um eine elektrische Verbindung zwischen den Kondensatoren (54,56) und dem Gehäuse herzustellen.

3. Durchführungsanschluß nach Anspruch 1, bei dem die bipolare Kondensatorscheibe (30) eine plattierten äußere Peripherie aufweist und eine Mehrzahl von Laminierungen (39,43,47), von der jede Laminierung einen dicken leitenden Film (40) auf einem isolierenden Substrat sowie erste und zweite Röhrenlöcher (26,28) aufweist, die durch die Laminierung hindurchgehen, wobei es mindestens zwei diskrete Typen (39,43,47) der Laminierung gibt, die entsprechend der Form des leitenden Filmes (40) darauf klassifiziert sind, wobei benachbarte Laminierungen (39,43,47) in keinem Fall vom gleichen Typ sind.

4. Anschluß nach Anspruch 3, bei dem ein erster Laminierungstyp (39) einen dicken leitenden Film (40) aufweist, der sich bis an die Peripheriekante der Laminierung (39) erstreckt aber einen Abstand zu den Röhrenlöchern (26,28) hat, und mit einem zweiten Laminierungstyp (43,47), der einen dicken leitenden Film (40) aufweist, der sich bis an die Kante nur eines der Röhrenlöcher (26,28) erstreckt und einen Abstand zu der Peripheriekante der Laminierung (43,47) aufweist.

5. Anschluß nach Anspruch 4, bei dem ein Paar der Laminierungen des zweiten Typs (43,47) auf gegenüberliegenden Seiten einer Laminierung des ersten Typs (39) angeordnet sind, wobei das Paar (43,47) so orientiert ist, daß es mit jeweils einem der ersten und zweiten Kontaktröhren (22,24) elektrischen Kontakt herstellt, wodurch separate Kondensatoren (56,58) zwischen den jeweils ersten und zweiten Kontaktröhren (22,24) und der Laminierung des ersten Typs (39) gebildet werden.

6. Anschluß nach Anspruch 4, bei dem die Laminierungen so angeordnet sind, daß sie verschachtelte parallel liegende Kondensatoren bilden, einen ersten solchen Kondensator (54) zwischen der ersten Kontaktröhre (22) und dem Gehäuse (12) und einen zweiten solchen Kondensator (56) zwischen der zweiten Kontaktröhre (24) und dem Gehäuse.

7. Anschluß nach Anspruch 4, wobei die bipolare Kondensatorscheibe (30) n Laminierungen des zweiten Typs (43,47) und n+1 Laminierungen des ersten Typs (39) aufweist, die verschachtelt zusammen mit n/2 Laminierungen des zweiten Typs (43,47) zusammen mit der ersten Kontaktröhre (22) verbunden sind, und wobei die verbleibenden Laminierungen des zweiten Typs (43,47) Zusammen mit der zweiten Kontaktröhre (24) und die Laminierungen des ersten Typs (39) zusammen mit einem gemeinsamen Referenzpunkt verbunden sind.

8. Anschluß nach Anspruch 2, bei dem die federnden leitenden Mittel (32) einen nicht geschlossenen Ring (32) aus einem dünnen metallischen Band (54) mit einer Mehrzahl von Streifen (56) aufweisen, deren Enden von der Ringoberfläche abstehen, um einen Kontakt zwischen der peripheren metallischen Oberfläche der Scheibe (30) und der inneren Oberfläche des Gehäuses (12) herzustellen.

9. Anschluß nach Anspruch 8, bei dem die Streifen (56) durch transversal verlaufender Einschnitte in das dünne metallische Band längs einer Kante davon erzeugt werden und wobei jeder Streifen leicht erdreht wird in bezug auf einen benachbarten nicht geschnittenen Teil des Bandes im Anschluß an den Streifen .

10. Anschluß nach Anspruch 10 oder 12, bei dem der Kontaktring (32) Nickel von einer Dicke von etwa 0,003 Zoll aufweist und jeder Streifen (56) im wesentlichen rechteckig ist mit einer Länge, die annähernd gleich 0,3 mal der Breite des Bandes (54) ist.

11. Anschluß nach einem der Ansprüche 3 bis 10, bei dem jede Kontaktröhre (23,24) ein Endstück (22a) an einem ersten Ende der Röhre mit einem ersten Durchmesser, einen Flansch (22b) an einem zweiten Ende der Röhre und ein Mittelstück (22c) zwischen dem Endstück (22a) und dem Flansch (22b) aufweist, wobei das Mittelstück (22e) einen zweiten Durchmesser aufweist, der größer ist als der erste Durchmesser und kleiner als die Durchmesser des ersten und zweiten Röhrenloches (26,28), um eine Einführung dahinein zu ermöglichen.

12. Anschluß nach Anspruch 11, bei dem das Mittelstück (22c) so deformiert ist, daß es von der Kreisform abweicht, um einen Preßsitz zu erzeugen, wenn es in das korrespondierende Röhrenloch (26,28) eingeführt wird.

## Revendications

1. Une borne de traversée bipolaire filtrée (10) comprenant :
une coquille tubulaire conductrice cylindrique (12);
un chapeau d'extrémité isolant (16) à travers lequel s'étendent des premier et second conducteurs électriques (18, 20) qui sont fixés hermétiquement dans le chapeau, ce chapeau d'extrémité (16) étant fixé hermétiquement à l'intérieur d'une première extrémité de la coquille (12);
des premier et second tubes métalliques de contact (22, 24), montés sur des extrémités des conducteurs (18, 20) à l'intérieur de la coquille (12); et
un disque de condensateur bipolaire feuilleté (30) s'ajustant dans une seconde extrémité de la coquille (12), dans une configuration dans laquelle les premier et second tubes de contact (22, 24) traversent ce disque (30), ce disque (30) définissant des condensateurs séparés (54, 56) entre la coquille et les premier et second tubes de contact respectifs (22, 24).

2. La borne de la revendication 1, comprenant en outre des moyens conducteurs élastiques (32) qui sont ajustés entre une périphérie extérieure du disque feuilleté (30) et une surface intérieure de la coquille (12) pour établir une connexion électrique entre les condensateurs (54, 56) et la coquille.

3. La borne de traversée de la revendication 1, dans laquelle le disque de condensateur bipolaire (30) comporte une circonférence extérieure métallisée et il comprend un ensemble de plaques (39, 43, 47), chaque plaque comprenant une pellicule conductrice épaisse (40) sur un substrat isolant et étant traversée par des premier et second trous (26, 28) de passage de tube, et il existe au moins deux catégories discrètes (39, 43, 47) de plaques, classées conformément à la forme de la pellicule conductrice (40) qu'elles portent, deux plaques (39, 43, 47) adjacentes n'étant jamais du même type.

4. La borne de la revendication 3, dans laquelle une première catégorie de plaque (39) comprend une pellicule conductrice épaisse (40) qui s'étend sur le bord périphérique de la plaque (39) mais est espacée des trous (26, 28) de passage de tube, et une seconde catégorie de plaque (43, 47) comprend une pellicule conductrice épaisse (40) qui s'étend jusqu'au bord de l'un seulement des trous (26, 28) de passage de tube et qui est espacée du bord périphérique de la plaque (43, 47).

5. La borne de la revendication 4, dans laquelle une paire de plaques de la seconde catégorie (43, 47) sont disposées de part et d'autre d'une plaque de la première catégorie (39), avec cette paire (43, 47) orientée de façon à établir un contact électrique avec des tubes respectifs parmi les premier et second tubes de contact (22, 24), pour former ainsi des condensateurs séparés (56, 58) entre les premier et second tubes de contact respectifs (22, 24) et la plaque de la première catégorie (39).

6. La borne de la revendication 4, dans laquelle les plaques sont disposées de façon à former des condensateurs à plaques parallèles entrelacées, un premier condensateur (54) étant formé entre le premier tube de contact (22) et la coquille (12), et un second condensateur (56) étant formé entre le second tube de contact (24) et la coquille.

7. La borne de la revendication 4, dans laquelle le disque de condensateur bipolaire(30) comprend n plaques du second type (43, 47) et n+1 plaques du premier type (39) entrelacées avec n/2 plaques du second type (43, 47) qui sont connectées ensemble au premier tube de contact (22), et les plaques restantes du second type (43, 47) étant connectées ensemble au second tube de contact (24), les plaques du premier type (39) étant connectées ensemble à un point de référence commun.

8. La borne de la revendication 2, dans laquelle les moyens conducteurs élastiques (32) consistent en un anneau ouvert (32) formé par une bande métallique mince (54) avec un ensemble de languettes (56) ayant des bords qui font saillie à partir des surfaces de l'anneau, pour établir le contact entre la surface métallique périphérique du disque (30) et la surface intérieure de la coquille (12).

9. La borne de la revendication 8, dans laquelle les languettes (56) sont formées par un ensemble de coupures transversales dans la bande métallique mince, le long d'un bord de celle-ci, chaque languette présentant une légère torsion par rapport à une partie non coupée adjacente de la bande dans une position contiguë à la languette.

10. la borne de la revendication 11 ou 12, dans laquelle l'anneau de contact (32) consiste en nickel ayant une épaisseur d'environ 76 micromètres, et chaque languette (56) est pratiquement carrée avec une longueur approximativement égale à 0,3 fois la largeur de la bande (54).

11. La borne de l'une quelconque des revendications 3 à 10, dans laquelle chaque tube de contact (22, 24) comprend une partie d'extrémité (22a) à une première extrémité du tube (12) ayant un premier diamètre, un collet (22b) à une seconde extrémité du tube, et une partie de corps (22c) entre la partie d'extrémité précitée (22a) et le collet (22b), cette partie de corps (22c) ayant un second diamètre qui est supérieur au premier diamètre et inférieur aux diamètres des premier et second trous de passage de tube (26, 28), pour permettre l'introduction dans ceux-ci.

12. La borne de la revendication 11, dans laquelle la partie de corps (22c) est déformée par rapport à une configuration circulaire, pour établir un ajustement serré lorsqu'elle est introduite dans un trou de passage de tube (26, 28) correspondant.
